(19) Europäisches Patentamt
European Patent Office
Office européen des brevets

(11) Veröffentlichungsnummer: **0 204 230 B1**

(12) **EUROPÄISCHE PATENTSCHRIFT**

(45) Veröffentlichungstag der Patentschrift: **22.07.92**

(51) Int. Cl.5: **A61K 31/415**, //A61K47/00, (A61K31/415,31:17)

(21) Anmeldenummer: **86107041.5**

(22) Anmeldetag: **23.05.86**

(54) **Azolderivate enthaltende Formulierungen sowie ihre Verwendung zur atraumatischen Nagelentfernung.**

(30) Priorität: **05.06.85 DE 3520098**

(43) Veröffentlichungstag der Anmeldung:
**10.12.86 Patentblatt 86/50**

(45) Bekanntmachung des Hinweises auf die Patenterteilung:
**22.07.92 Patentblatt 92/30**

(84) Benannte Vertragsstaaten:
**AT BE CH DE FR GB IT LI NL SE**

(56) Entgegenhaltungen:
**EP-A- 0 064 830**
**EP-A- 0 106 173**
**US-A- 4 070 451**

**DIALOG 05511807 Medline 85127807, Juni 1985; S. NOLTING: "Non-traumatic removal of the nail and simultaneous treatment of onychomycosis", & DERMATOLOGICA 1984, 169, SUPPL. 1, 117-120**

**DIALOG 04975636 Medline 83208636, September 1983; B. MONCADA et al.: "Treatment of onychomycosis with ketoconazole and nonsurgical avulsion of the affected nail", & CUTIS APRIL 1983, 31(4), 438-440**

**DIALOG 04771547 Medline 83004547, Januar 1983; O. ROLLMAN: "Treatment of onychomycosis by partial nail avulsion and topical miconazole", & DERMATOLOGICA Juli 1982, 165, C1, 54-61**

(73) Patentinhaber: **BAYER AG**

**W-5090 Leverkusen 1 Bayerwerk(DE)**

(72) Erfinder: **Stettendorf, Sigrid, Dr.**
**Oberer Grifflenberg 87**
**W-5600 Wuppertal 1(DE)**
Erfinder: **Adams, Karlheinz, Dr.**
**Haferkamp 6**
**W-5000 Köln 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft Azolderivate enthaltende Formulierungen sowie ihre Verwendung zur atraumatischen Nagelentfernung und ein Verfahren zur Herstellung dieser Formulierungen.

Als Azolderivate seien Bifonazol der Formel

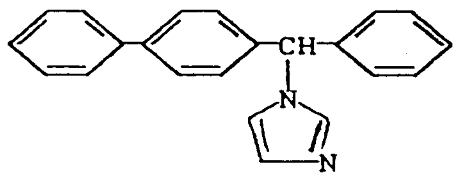

und Clotrimazol der Formel

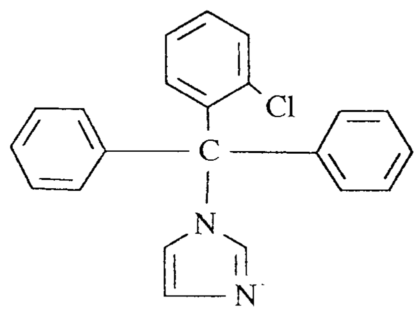

genannt.

Aus US-A-4070451, EP-A-0106173, Dermatologica 1984, 169, Suppl 1, 117 - 120, ibid. 1982, 165, C1, 54 - 61 sowie Cutis, 4, 1983, 31, 38 - 40 sind Formulierungsbasen bzw. Azolderivate enthaltende Formulierungen bekannt geworden. Diese sind jedoch zur atraumatischen Nagelentfernung entweder nicht geeignet oder die Formulierungen sind nicht hinreichend stabil.

Es wurde gefunden, daß Formulierungen, die diese Azolderivate in Mengen von 0,05 - 1,5, vorzugsweise von 0,1 - 1, insbesondere von einem Gewichtsteil(en) enthalten und weiterhin aus

a) 40 Gew.-Teilen Harnstoff
b) 20 Gew.-Teilen wasserfreiem Wollfett,
c) 5 Gew.-Teilen gebleichtem Wachs sowie
d) 34 Gew-Teilen weißer Vaseline bestehen,

eine hervorragende Lagerstabilität aufweisen und zur atraumatischen Nagelentfernung sehr gut geeignet sind.

Die erfindungsgemäßen Formulierungen können hergestellt werden, indem man das wasserfreie Wollfett, das gebleichte Wachs sowie die weiße Vaseline bei erhöhter Temperatur, beispielsweise bei 50 - 80 °C, mischt und anschließend das Azolderivat und den Harnstoff zugibt. Vorzugsweise wird Harnstoff zuletzt zugegeben.

Die erfindungsgemäße Formulierung kann zur atraumatischen Nagelentfernung bei Onychomykosen bei Mensch und Tier eingesetzt werden.

Die Azolderivate entfalten ihre antimykotische Wirkung in der erfindungsgemäßen Formulierung durch eine verbesserte Penetration am durch Mykosen infiziertem Nagel für die Zeit bis zur Nagelentfernung. Harnstoff bewirkt aufgrund seiner hydratisierenden und keratolytischen Eigenschaften eine Auflockerung und nachfolgende atraumatische Ablösung der infizierten Nagelplatte. Der folgende Versuch verdeutlicht dies.

Die beispielsgemäße Zusammensetzung wird alle 24 Stunden auf den pilzbefallenen Nagel aufgetragen und unter Okklusivverband 24 Stunden belassen. Unter diesem Vorgehen ist der pilzinfizierte Nagel in der Regel in einem Zeitraum zwischen 6 bis 10 Tagen atraumatisch ablösbar.

Beispiel

| | |
|---|---|
| Bifonazol | 1 % |
| Harnstoff | 40 % |
| Wollfett wasserfrei | 20 % |
| gebleichtes Wachs | 5 % |
| Vaseline weiß | 34 % |
| | 100 % |

**Patentansprüche**

1. Formulierungen, dadurch gekennzeichnet, daß sie aus 0,05 - 1,5 Gew.-Teilen Bifonazol und/oder Clotrimazol, 40 Gew.-Teilen Harnstoff, 20 Gew.-Teilen wasserfreiem Wollfett, 5 Gew.-Teilen gebleichtem Wachs sowie 34 Gew.-Teilen weißer Vaseline bestehen.

2. Formulierungen gemäß Anspruch 1 zur atraumatischen Nagelentfernung.

3. Verfahren zur Herstellung von Formulierungen gemäß Anspruch 1 dadurch gekennzeichnet, daß man das wasserfreie Wollfett, das gebleichte Wachs sowie die weiße Vaseline bei erhöhter Temperatur mischt und anschließend das Bifonazol und/oder Clotrimazol sowie den Harnstoff zugibt.

**Claims**

1. Formulations, characterized in that they consist of 0.05 - 1.5 parts by weight of bifonazole and/or clotrimazole, 40 parts by weight of urea, 20 parts by weight of anhydrous wool fat, 5 parts by weight of bleached wax and 34 parts by weight of white petroleum jelly.

2. Formulations according to Claim 1 for atraumatic nail removal.

3. Process for the preparation of formulations according to Claim 1, characterised in that the anhydrous wool fat, the bleached wax and the white petroleum jelly are mixed at elevated temperature and the bifonazole and/or clotrimazole and the urea are then added.

**Revendications**

1. Formulations caractérisées en ce qu'elles consistent en 0,05-1,5 partie en poids de bifonazol et/ou de clotrimazol, 40 parties en poids d'urée, 20 parties en poids de suint anhydre, 5 parties en poids de cire blanchie et 34 parties en poids de vaseline blanche.

2. Formulations selon la revendication 1 pour l'ablation atraumatique des ongles.

3. Procédé pour la fabrication de formulations selon la revendication 1, caractérisé en ce que l'on mélange à température élevée le suint anhydre, la cire blanchie et la vaseline blanche et ensuite on ajoute le bifonazol et/ou le clotrimazol et l'urée.